(19) Europäisches Patentamt
European Patent Office
Office européen des brevets

(11) **EP 2 012 843 B1**

(12) **EUROPÄISCHE PATENTSCHRIFT**

(45) Veröffentlichungstag und Bekanntmachung des Hinweises auf die Patenterteilung:
**27.10.2021 Patentblatt 2021/43**

(21) Anmeldenummer: **07724412.7**

(22) Anmeldetag: **20.04.2007**

(51) Int Cl.:
*A61L 15/60* (2006.01)      *C08F 8/44* (2006.01)
*C08J 3/24* (2006.01)

(86) Internationale Anmeldenummer:
**PCT/EP2007/003475**

(87) Internationale Veröffentlichungsnummer:
**WO 2007/121937 (01.11.2007 Gazette 2007/44)**

(54) **WASSERABSORBIERENDES POLYMERGEBILDE MIT VERBESSERTER PERMEABILITÄT UND ABSORPTION UNTER DRUCK SOWIE VERFAHREN ZUR HERSTELLUNG**

WATER-ABSORBENT POLYMER STRUCTURE WITH IMPROVED PERMEABILITY AND ABSORPTION UNDER PRESSURE AND PROCESS OF PREPARATION

STRUCTURE POLYMÉRIQUE ABSORBANT L'EAU À PERMÉABILITÉ ET ABSORPTION SOUS PRESSION AMÉLIORÉES AINSI QUE LE PROCÉDÉ DE FABRICATION

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **21.04.2006 DE 102006019157**
**21.04.2006 PCT/EP2006/003695**
**21.04.2006 PCT/EP2006/003694**
**21.04.2006 PCT/EP2006/003696**
**12.08.2006 DE 102006037983**

(43) Veröffentlichungstag der Anmeldung:
**14.01.2009 Patentblatt 2009/03**

(60) Teilanmeldung:
**18206625.8**

(73) Patentinhaber: **Evonik Operations GmbH**
**45128 Essen (DE)**

(72) Erfinder:
• WALDEN, Mirko
  45699 Herten (DE)
• SCHMIDT, Harald
  47918 Tönisvorst (DE)
• TENI, Rainer
  47447 Moers (DE)
• REIMANN, Armin
  47877 Willich (DE)
• FURNO, Franck
  40545 Düsseldorf (DE)
• ISSBERNER, Jörg
  47877 Willich-Neersen (DE)
• HERBE, Peter
  47623 Kevelaer (DE)
• NIELINGER, Ursula
  47802 Krefeld (DE)
• KEUP, Michael
  45139 Essen (DE)

(74) Vertreter: **Evonik Patent Association et al**
**c/o Evonik Industries AG**
**IP Management**
**Bau 1042A/PB 15**
**Paul-Baumann-Straße 1**
**45772 Marl (DE)**

(56) Entgegenhaltungen:
EP-A- 1 616 581          WO-A-2006/111402
WO-A2-2004/037903     DE-A1- 19 909 653
US-A- 5 973 042

• None

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren zur Herstellung wasserabsorbierender Polymergebilde, die durch dieses Verfahren erhältlichen wasserabsorbierenden Polymergebilde.

Superabsorber sind wasserunlösliche, vernetzte Polymere, die in der Lage sind, unter Quellung und Ausbildung von Hydrogelen große Mengen an wässrigen Flüssigkeiten, insbesondere Körperflüssigkeiten, vorzugsweise Urin oder Blut, aufzunehmen und unter einem bestimmten Druck zurückzuhalten. Durch diese charakteristischen Eigenschaften finden diese Polymere hauptsächlich Anwendung bei der Einarbeitung in Sanitärartikeln, wie beispielsweise Babywindeln, Inkontinenzprodukten oder Damenbinden.

Die Herstellung der Superabsorber erfolgt in der Regel durch die radikalische Polymerisation säuregruppentragender Monomere in Gegenwart von Vernetzern. Dabei lassen sich durch die Auswahl der Monomerzusammensetzung, der Vernetzer sowie der Polymerisationsbedingungen und der Verarbeitungsbedingungen für das nach der Polymerisation erhaltene Hydrogel Polymere mit unterschiedlichen Absorbereigenschaften herstellen. Weitere Möglichkeiten bietet die Herstellung von Pfropfpolymerisaten, beispielsweise unter Verwendung chemisch modifizierter Stärke, Cellulose und Polyvinylalkohol nach DE-OS 26 12 846.

Bei den gegenwärtig kommerziell verfügbaren Superabsorbern handelt es sich im wesentlichen um vernetzte Polyacrylsäuren oder vernetzte Stärke-Acrylsäure-Pfropfpolymerisate, bei denen die Carboxylgruppen teilweise mit Natronlauge oder Kalilauge neutralisiert sind.

Aus ästhetischen Gründen und aus Umweltaspekten besteht zunehmend die Tendenz, die Sanitärartikel immer kleiner und dünner zu gestalten. Um ein gleichbleibendes Gesamtretentionsvermögen der Sanitärartikel zu gewährleisten, kann dieser Anforderung nur durch Reduktion des Anteils an großvolumigen Fluff entsprochen werden. Hierdurch fallen dem Superabsorber weitere Aufgaben hinsichtlich Transport und Verteilung von Flüssigkeit zu, die sich als Permeabilitätseigenschaften zusammenfassen lassen.

Unter Permeabilität versteht man bei Superabsorbermaterialien die Fähigkeit, im gequollenen Zustand zugegebene Flüssigkeiten zu transportieren und dreidimensional zu verteilen. Dieser Prozess läuft im gequollenen Superabsorbergel über kapillaren Transport durch Zwischenräume zwischen den Gelpartikeln ab. Ein Flüssigkeitstransport durch gequollene Superabsorberpartikel selbst folgt den Gesetzen der Diffusion und ist ein sehr langsamer Prozess, der in der Nutzungssituation des Sanitärartikels keine Rolle bei der Verteilung der Flüssigkeit spielt. Bei Superabsorbermaterialien, die einen kapillaren Transport aufgrund mangelnder Gelstabilität nicht bewerkstelligen können, wurde durch Einbetten dieser Materialien in eine Fasermatrix eine Separation der Partikel voneinander unter Vermeidung des Gel-Blocking-Phänomens sichergestellt. In Windelkonstruktionen neuer Generation befindet sich in der Absorberschicht nur wenig oder überhaupt kein Fasermaterial zur Unterstützung des Flüssigkeitstransports. Die hier verwendeten Superabsorber müssen demnach eine ausreichend hohe Stabilität im gequollenen Zustand besitzen, damit das gequollene Gel noch eine ausreichende Menge an kapillaren Räumen besitzt, durch die Flüssigkeit transportiert werden kann.

Um Superabsorbermaterialien mit hoher Gelstabilität zu erhalten, kann einerseits der Grad der Vernetzung des Polymers angehoben werden, was zwangsläufig eine Verminderung der Quellfähigkeit und des Retentionsvermögens zur Folge hat. Eine optimierte Kombination von verschiedenen Vernetzern und Comonomeren, wie in DE 196 46 484 beschrieben, vermag die Permeabilitätseigenschaften zwar verbessern, nicht aber auf ein Niveau, das beispielsweise den Einbau einer gegebenenfalls nur aus Superabsorbern bestehende Schicht in eine Windelkonstruktion erlaubt. Weiterhin können Methoden zur Nachbehandlung der Oberfläche von Polymerpartikeln zur Verbesserung der Superabsorbereigenschaften zum Einsatz kommen. Als Oberflächenbehandlung sind beispielsweise Nachvernetzung des absorbierenden Polymergebildes an der Oberfläche, das in Kontakt bringen der Oberfläche mit anorganischen Verbindungen oder aber die Nachvernetzung der Oberfläche in Gegenwart anorganischer Verbindungen aus dem Stand der Technik bekannt.

So offenbaren DE 199 09 653 A1 und DE 199 09 838 A1 pulverförmige, an der Oberfläche nachvernetzte, Wasser, wässrige oder seröse Flüssigkeiten oder Blut absorbierende Polymerisate, welche auf säuregruppen-tragenden Monomeren basieren und welche mit einem Oberflächennachvernetzungsmittel und vorzugsweise Aluminiumsulfat in wässriger Lösung beschichtet und nachvernetzt worden ist. Die in diesem Stand der Technik offenbarten Polymerisate weisen gegenüber herkömmlichen Polymerisaten vorteilhafte Absorptionseigenschaften, insbesondere eine hohe Permeabilität auf.

DE 102 49 821 A1 beschreibt ebenfalls pulverförmige, an der Oberfläche nachvernetzte, wasserabsorbierende Polymergebilde mit hoher Permeabilität, die durch Oberflächenbehandlung von unbehandelten, wasserabsorbierenden Polymergebilden mit einer Mischung aus einem Vernetzer und einem anorganischen Sol, beispielsweise Kieselsäuresol, erhalten wurden.

Der Nachteil der aus dem Stand der Technik bekannten Oberflächenmodifizierungsmaßnahmen, insbesondere der Behandlung wasserabsorbierender Polymergebilde mit anorganischen Salzen oder anorganischen Solen zum Zwecke der Permeabilitätsverbesserung besteht jedoch insbesondere darin, dass die beobachtete Erhöhung der Permeabilität häufig auch mit einer signifikanten Verminderung des Absorptionsvermögens unter Druck einhergeht. Diese Verminderung des Absorptionsvermögens unter Druck führt oft zu einer erhöhten Leckage-Neigung der Hygieneartikel nach dem einmaligen und insbesondere mehrfachen Einessen durch den Verwender.

Der vorliegenden Erfindung lag die Aufgabe zugrunde, die sich aus dem Stand der Technik ergebenden Nachteile zu überwinden.

Insbesondere lag der vorliegenden Erfindung die Aufgabe zugrunde, Superabsorber bereitzustellen, der zum einen den Transport von wässrigen Flüssigkeiten wie Urin in Hygieneartikeln wie Sauglagen bzw. Cores, insbesondere für Windeln, mit hohen Superabsorberkonzentrationen fördert und so den Tragekomfort dieser Hygienartikel zu steigern.

Weiterhin lag der vorliegenden Erfindung die Aufgabe zugrunde, ein Verfahren anzugeben, mit dem derart vorteilhafte Superabsorber sowie ein Verbund beinhaltend diese Superabsorber hergestellt werden können.

Auch lag der vorliegenden Erfindung die Aufgabe zugrunde, möglichst dünne Hygieneartikel mit einem hohen Gehalt an wasserabsorbierenden Polymergebilden bereitzustellen, welche durch gute Gebrauchseigenschaften gekennzeichnet sind.

Einen Beitrag zur Lösung der vorstehend genannten Aufgaben liefert ein Verfahren zur Herstellung eines wasserabsorbierenden Polymergebildes nach Anspruch 1.

Völlig überraschend, jedoch nicht minder vorteilhaft wurde festgestellt, dass sich der Abfall der Absorption unter einer Druckbelastung, welcher häufig bei der Oberflächenmodifizierung wasserabsorbierender Polymergebilde mit permeabilitätssteigernden Mitteln beobachtet wird, durch die Verwendung von Salzen als permeabilitätssteigernde Mittel, umfassend ein zwei- oder höherwertiges Kation eines Metalls sowie mindestens eine organische Base als Anion, vermindern lässt.

"Unbehandelt" im Sinne der vorliegenden Erfindung bedeutet, dass die im Verfahrenschritt i) bereitgestellten, wasserabsorbierenden Polymergebilde noch nicht mit dem Salz umfassend ein zwei- oder höherwertiges Kation eines Metalls sowie mindestens eine organische Base als Anion in Kontakt gebracht worden sind. Die Bezeichnung "unbehandelt" schließt hingegen nicht aus, dass die wasserabsorbierenden Polymergebilde mittels anderer Oberflächenmodifizierungsmaßnahmen, wie etwa der Oberflächennachvernetzung, modifiziert sein können.

Als im Verfahrensschritt i) bereitgestellte unbehandelte, wasserabsorbierende Polymergebilde sind Fasern, Schäume oder Teilchen bevorzugt, wobei Fasern und Teilchen bevorzugt, aber nicht als solche beansprucht sind. Teilchen sind besonders bevorzugt aber nicht als solche beansprucht.

Polymerfasern sind so dimensioniert, dass sie in oder als Garne für Textilien und auch direkt in Textilien eingearbeitet werden können. Polymerfasern können eine Länge im Bereich von 1 bis 500 mm, oder 2 bis 500 mm oder 5 bis 100 mm und einen Durchmesser im Bereich von 1 bis 200 Denier, oder 3 bis 100 Denier oder 5 bis 60 Denier besitzen.

Polymerteilchen sind so dimensioniert, dass sie eine mittlere Teilchengröße gemäß ERT 420.2-02 im Bereich von 10 bis 3000 $\mu$m, oder 20 bis 2000 $\mu$m oder 150 bis 850 $\mu$m oder 150 bis 600 $\mu$m aufweisen. Der Anteil der Polymerteilchen mit einer Partikelgröße in einem Bereich von 300 bis 600 $\mu$m beträgt mindestens 30 Gew.-%, oder mindestens 40 Gew.-% oder mindestens 50 Gew.-%, bezogen auf das Gesamtgewicht der nachvernetzten, wasserabsorbierenden Polymerteilchen.

In einer bevorzugten Ausführungsform der im Verfahrensschritt i) bereitgestellten, wasserabsorbierenden Polymergebilde basieren diese auf

($\alpha$1) 20-99,999 Gew.-%, bevorzugt 55-98,99 Gew.-% und besonders bevorzugt 70-98,79 Gew.-% polymerisierten, ethylenisch ungesättigten, säuregruppen-tragenden Monomeren oder deren Salze oder polymerisierten, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,

(a2) 0-80 Gew.-%, vorzugsweise 0-44,99 Gew.-% und besonders bevorzugt 0,1-44,89 Gew.-% polymerisierten, monoethylenisch ungesättigten, mit ($\alpha$1) copolymerisierbaren Monomeren,

(a3) 0,001-5 Gew.-%, vorzugsweise 0,01-3 Gew.-% und besonders bevorzugt 0,01-2,5 Gew.-% eines oder mehrerer Vernetzer,

(a4) 0-30 Gew.-%, vorzugsweise 0-5 Gew.-% und besonders bevorzugt 0,1-5 Gew.-% eines wasserlöslichen Polymeren,

(a5) 0-20 Gew.-%, vorzugsweise 2,5-15 Gew.-% und besonders bevorzugt 5-10 Gew.-% Wasser, sowie

(a6) 0-20 Gew.-%, vorzugsweise 0-10 Gew.-% und besonders bevorzugt 0,1-8 Gew.-% eines oder mehrerer Hilfsmittel, wobei die Summe der Gewichtsmengen ($\alpha$1) bis (a6) 100 Gew.-% beträgt.

Die monoethylenisch ungesättigten, säuregruppenhaltigen Monomere ($\alpha$1) können teilweise oder vollständig, bevorzugt teilweise neutralisiert sein. Vorzugsweise sind die monoethylenisch ungesättigten, säuregruppenhaltigen Monomere zu mindestens 25 Mol-%, besonders bevorzugt zu mindestens 50 Mol-% und darüber hinaus bevorzugt zu 50-80 Mol-% neutralisiert. In diesem Zusammenhang wird auf DE 195 29 348 A1 verwiesen. Die Neutralisation kann teilweise oder ganz auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak und Alkalimetallhydroxiden, besonders bevorzugt mit Natriumhydroxid und mit Ammoniak.

Ferner können bei einem Polymer die freien Säuregruppen überwiegen, so dass dieses Polymer einen im sauren Bereich liegenden pH-Wert aufweist. Dieses saure wasserabsorbierende Polymer kann durch ein Polymer mit freien basischen Gruppen, vorzugsweise Amingruppen, das im Vergleich zu dem sauren Polymer basisch ist, mindestens teilweise neutralisiert werden. Diese Polymere werden in der Literatur als "Mixed-Bed Ion-Exchange Absorbent Polymers" (MBIEA-Polymere) bezeichnet und sind unter anderem in der WO 99/34843 A1 offenbart. In der Regel stellen MBIEA-Polymere eine Zusammensetzung dar, die zum einen basische Polymere, die in der Lage sind, Anionen auszutauschen, und andererseits ein im Vergleich zu dem basischen Polymer saures Polymer, das in der Lage ist, Kationen auszutauschen, beinhalten. Das basische Polymer weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin und dergleichen, sowie deren sekundäre oder tertiäre Aminderivate.

Bevorzugte ethylenisch ungesättigte, säuregruppenhaltige Monomere ($\alpha$1) sind vorzugsweise diejenigen Verbindungen, die in der WO 2004/037903 A2 als ethylenisch ungesättigte, säuregruppenhaltige Monomere ($\alpha$1) genannt werden. Besonders bevorzugte ethylenisch ungesättigte, säuregruppenhaltige Monomere ($\alpha$1) sind Acrylsäure und Methacrylsäure, wobei Acrylsäure am meisten bevorzugt ist.

Gemäß einer Ausführungsform des erfindungsgemäßen Verfahrens werden unbehandelte, wasserabsorbierende Polymergebilde eingesetzt, bei denen die monoethylenisch ungesättigten, mit ($\alpha$1) copolymerisierbare Monomere (a2) Acrylamide, Methacrylamide oder Vinylamide sind.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino-(meth)acrylamid, Dimethyl(meth)acrylamid oder Diethyl(meth)acrylamid. Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Gemäß einer Ausführungsform des erfindungsgemäßen Verfahrens werden wasserabsorbierende Polymergebilde eingesetzt, bei denen die monoethylenisch ungesättigten, mit ($\alpha$1) copolymerisierbare Monomere (a2) wasserlösliche Monomere sind. In diesem Zusammenhang sind insbesondere Alkoxypolyalkylenoxid(meth)acrylate wie Methoxypolyethylenglykol(meth)acrylate bevorzugt.

Des Weiteren sind als monoethylenisch ungesättigte, mit ($\alpha$1) copolymerisierbaren Monomere (a2) in Wasser dispergierbare Monomere bevorzugt. Als in Wasser dispergierbare Monomere sind Acrylsäureester und Methacrylsäureester, wie Methyl(meth)acrylat, Ethyl(meth)acrylat, Propyl(meth)acrylat oder Butyl(meth)-acrylat bevorzugt.

Die monoethylenisch ungesättigten, mit ($\alpha$1) copolymerisierbaren Monomere (a2) umfassen weiterhin Methylpolyethylenglykolallylether, Vinylacetat, Styrol und Isobutylen.

Als Vernetzer (a3) werden vorzugsweise diejenigen Verbindungen eingesetzt, die in der WO 2004/037903 A2 als Vernetzer (a3) genannt werden. Unter diesen Vernetzern sind wasserlösliche Vernetzer besonders bevorzugt. Am meisten bevorzugt sind dabei N,N'-Methylenbisacrylamid, Polyethylenglykoldi-(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Als wasserlösliche Polymere (a4) können in den Polymergebilden wasserlösliche Polymere, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsmittel (a6) sind vorzugsweise Stellmittel, Geruchsbinder, oberflächenaktive Mittel oder Antioxidatien sowie diejenigen Additive, die zur Herstellung der Polymergebilde eingesetzt wurden (Initiatoren usw.) in den Polymergebilden enthalten.

In einer besonderen Ausführungsform der im Verfahrensschritt i) bereitgestellten, wasserabsorbierenden Polymergebilde basieren diese zu mindestens 50 Gew.-%, vorzugsweise zu mindestens 70 Gew.-% und darüber hinaus bevorzugt zu mindestens 90 Gew.-% auf carboxylatgruppen-tragenden Monomeren. Es ist erfindungsgemäß weiterhin bevorzugt, dass die Komponente ($\alpha$1) zu mindestens 50 Gew.-%, vorzugsweise zu mindestens 70 Gew.-% aus Acrylsäure besteht, die vorzugsweise zu mindestens 20 Mol-%, besonders bevorzugt zu mindestens 50 Mol-% und darüber hinaus bevorzugt in einem Bereich von 60 bis 85 Mol-% neutralisiert ist.

Aus den vorgenannten Monomeren, Comonomeren, Vernetzern, wasserlöslichen Polymeren und Hilfsstoffen lassen sich die unbehandelten, wasserabsorbierenden Polymergebilde durch verschiedene Polymerisationsweisen herstellen. Beispielsweise sind in diesem Zusammenhang Massepolymerisation, die vorzugsweise in Knetreaktoren wie Extrudern erfolgt, Lösungspolymerisation, Spraypolymerisation, inverse Emulsionspolymerisation und inverse Suspensionspolymerisation zu nennen.

Bevorzugt wird die Lösungspolymerisation in Wasser als Lösungsmittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 40 20 780, DE 42 44 548, DE 43 23 001, DE 43 33 056, DE 44 18 818.

Die Polymerisation wird wie allgemein üblich durch einen Initiator ausgelöst. Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der oben genannten Art durch Einwirkung energiereicher Strahlung in Gegenwart von Photoinitiatoren ausgelöst werden. Polymerisationsinitiatoren können in einer Lösung erfindungsgemäßer Monomere gelöst oder dispergiert enthalten sein. Als Initiatoren kommen sämtliche dem Fachmann bekannte in Radikale zerfallende Verbindungen in Betracht. Hierunter fallen insbesondere diejenigen Initiatoren, die bereits in der WO 2004/037903 A2 als mögliche Initiatoren genannt werden.

Besonders bevorzugt wird zur Herstellung der wasserabsorbierenden Polymergebilde ein Redoxsystem bestehend aus Wasserstoffperoxid, Natriumperoxodisulfat und Ascorbinsäure eingesetzt.

Auch die inverse Suspensions- und Emulsionspolymerisation kann zur Herstellung der Polymergebilde angewendet werden. Gemäß diesen Prozessen wird eine wässrige, teilneutralisierte Lösung der Monomeren ($\alpha$1), und ($\alpha$2), gegebenenfalls beinhaltend wasserlösliche Polymere und Hilfsstoffe, mit Hilfe von Schutzkolloiden und/oder Emulgatoren in einem hydrophoben, organischen Lösungsmittel dispergiert und durch Radikalinitiatoren die Polymerisation gestartet. Die Vernetzer sind entweder in der Monomerlösung gelöst und werden mit dieser zusammen dosiert oder aber separat und gegebenenfalls während der Polymerisation zugefügt. Gegebenenfalls erfolgt die Zugabe eines wasserlöslichen Polymeren (a4) als Pfropfgrundlage über die Monomerlösung oder durch direkte Vorlage in die Ölphase. Anschließend wird das Wasser azeotrop aus dem Gemisch entfernt und das Polymerisat abfiltriert.

Weiterhin kann sowohl bei der Lösungspolymerisation als auch bei der inversen Suspensions- und Emulsionspolymerisation die Vernetzung durch Einpolymerisation des in der Monomerlösung gelösten polyfunktionellen Vernetzers und/oder durch Reaktion geeigneter Vernetzer mit funktionellen Gruppen des Polymeren während der Polymerisationsschritte erfolgen. Die Verfahren sind beispielsweise in den Veröffentlichungen US 4,340,706, DE 37 13 601, DE 28 40 010 und WO 96/05234 A1 beschrieben.

Die bei der Lösungspolymerisation oder der inversen Suspensions- und Emulsionspolymerisation nach der Polymerisation erhaltenen Hydrogele werden in einem weiteren Verfahrenschritt getrocknet.

Insbesondere im Falle der Lösungspolymerisation ist es jedoch bevorzugt, dass die Hydrogele vor der Trocknung zunächst zerkleinert werden. Dieses Zerkleinern erfolgt durch dem Fachmann bekannte Zerkleinerungsvorrichtungen, wie etwa einem Fleischwolf.

Die Trocknung des Hydrogels erfolgt vorzugsweise in geeigneten Trocknern oder Öfen. Beispielhaft seien Drehrohröfen, Wirbelbetttrockner, Tellertrockner, Paddeltrockner oder Infrarottrockner genannt. Weiterhin ist es erfindungsgemäß bevorzugt, dass die Trocknung des Hydrogels bis zu einem Wassergehalt von 0,5 bis 25 Gew.-%, vorzugsweise von 1 bis 10 Gew.-% erfolgt, wobei die Trocknungstemperaturen üblicherweise in einem Bereich von 100 bis 200°C liegen.

Die nach dem Trocknen erhaltenen wasserabsorbierenden Polymergebilde können, insbesondere dann, wenn sie durch Lösungspolymerisation erhalten wurden, in einem weiteren Verfahrensschritt noch zermahlt und auf eine die eingangs genannte Wunschkorngröße abgesiebt werden. Das Zermahlen der getrockneten, wasserabsorbierenden Polymergebilde erfolgt vorzugsweise in geeigneten, mechanischen Zerkleinerungsvorrichtungen, wie etwa einer Kugelmühle.

In einer bevorzugten Ausführungsform zeigt das im Verfahrensschritt i) des erfindungsgemäßen Verfahren bereitgestellte, unbehandelte absorbierende Polymergebilde mindestens eine der folgenden Eigenschaften (ERT = *EDANA Recommended Test*):

(A) die maximale Aufnahme gemäß ERT 440.2-02 (im Falle von Partikeln bestimmt für die gesamte Partikelgrößenfraktion) von 0,9 Gew.-%er NaCl-Lösung liegt in einem Bereich von mindestens 10 bis 1000 g/g, vorzugsweise im Bereich von 20 bis 500 g/g und darüber hinaus bevorzugt im Bereich von 50 bis 100 g/g,

(B) der gemäß ERT 470.2-02 (im Falle von Partikeln bestimmt für die gesamte Partikelgrößenfraktion) extrahierbare Anteil nach 16 Stunden beträgt weniger als 30 Gew.-%, vorzugsweise weniger als 20 Gew.-% und darüber hinaus bevorzugt weniger als 15 Gew.-%, jeweils bezogen auf das unbehandelte, wasserabsorbierende Polymergebilde,

(C) die Schüttdichte gemäß ERT 460.2-02 (im Falle von Partikeln bestimmt für die gesamte Partikelgrößenfraktion) liegt im Bereich von 300 bis 1000 g/l, vorzugsweise im Bereich von 400 bis 900 g/l und darüber hinaus bevorzugt von 500 bis 800 g/l,

(D) der pH-Wert gemäß ERT 400.2-02 (im Falle von Partikeln bestimmt für die gesamte Partikelgrößenfraktion) von 1 g des wasserabsorbierenden Polymervorprodukts in 1 l Wasser liegt im Bereich von 4 bis 10, vorzugsweise im Bereich von 4,5 bis 9 und darüber hinaus bevorzugt im Bereich von 5 bis 8,

(E) die nach ERT 442.2-02 (im Falle von Partikeln für die gesamte Partikelfraktion) bestimmte Absorption gegen einen Druck von 0,3 psi liegt in einem Bereich von 10 bis 26 g/g, vorzugsweise in einem Bereich von 13 bis 25 g/g und am meisten bevorzugt in einem Bereich von 13,5 bis 24 g/g;

(F) die nach ERT 441.2-02 (im Falle von Partikeln für die gesamte Partikelfraktion) bestimmte als CRC bezeichnete Retention liegt in einem Bereich von 20 bis 50 g/g, vorzugsweise in einem Bereich von 25 bis 45 g/g und am meisten bevorzugt in einem Bereich von 27 bis 40 g/g.

Gemäß einer besonderen Ausführungsform des erfindungsgemäßen Verfahrens werden im Verfahrensschritt i) Polymergebilde bereitgestellt, die durch die folgenden Eigenschaften bzw. Eigenschaftskombinationen gekennzeichnet sind: (A), (B), (C), (D), (E), (F), (A)(E), (B)(E), (C)(E), (D)(E), (E)(F), (B)(E), (B)(F), (E)(F)(G), wobei (D), (E), (F) und (E)(F) und (D)(E)(F) am meisten bevorzugt sind und (D)(E)(F) darüber hinaus bevorzugt ist.

Im Verfahrensschritt ii) des erfindungsgemäßen Verfahrens werden die im Verfahrensschritt i) bereitgestellten unbehandelten, wasserabsorbierenden Polymergebilde mit einem Salz umfassend ein zwei- oder höherwertiges Kation eines Metalls sowie mindestens eine organische Base als Anion in Kontakt gebracht.

Gemäß des erfindungsgemäßen Verfahrens wird im Verfahrensschritt ii) als Salz ein Salz eingesetzt, welches Aluminiumlactat beinhaltet. Vorzugsweise basiert dieses Salz zu mindestens 50 Gew.-%, darüber hinaus bevorzugt zu mindestens 75 Gew.-% und am meisten bevorzugt zu 100 Gew.-% auf Aluminiumlactat. Es ist weiterhin bevorzugt, dass neben dem Aluminiumlactat noch ein oder zwei oder mehrere weitere Kationen vorliegen. Dieses sind vorzugsweise ausgewählt aus der Gruppe bestehend aus: ein-, zwei- oder höherwertige Kation eines Metalls, wiederum ausgewählt aus der Gruppe bestehend aus $Mg^{2+}$, $Ca^{2+}$, $Ba^{2+}$, $Al^{3+}$, $Fe^{2+}$, $Fe^{3+}$, $Ga^{3+}$, $Ti^{4+}$, $Zr^{4+}$, $Cu^{2+}$ und $Zn^{2+}$. Zudem können neben den Aluminiumlactat in dem Salz auch weitere Anionen vorliegen. Hierbei sind die vorstehend beschriebenen Anionen bevorzugt. Ferner können neben Aluminiumlactat auch Oxide bzw. Mischoxide weiterer Metalle vorliegen, wobei die Oxide der in diesem Abschnitt genannten Metallionen bevorzugt sind. So kann es im Zusammenhang mit der einer das Salz beinhaltenden Lösung bevorzugt sein, dass diese zudem ein Alkali- oder Erdalkali-, vorzugsweise ein Alkalisalz eines der vorstehend genannten Anionen, vorzugsweise das hauptsächlich in der Lösung vorliegende Anion des Hauptsalzes, als Nebensalz beinhaltet. Hierunter fallen vorzugsweise Lithium- und Natriumlactat. Vorzugsweise beträgt die Menge des Nebensalzes 0,001 bis 25 Gew.-%, bevorzugt 0,01 bis 17 Gew.-% und besonders bevorzugt 0,1 bis 12 Gew.-%, jeweils bezogen auf das Hauptsalz.

In einer Ausgestaltung des erfindungsgemäßen Verfahrens ist es bevorzugt, dass ein weiteres Anion vorhanden ist, das von der organischen Base verschieden ist. Hierbei handelt es sich vorzugsweise um eine anorganische Base. Diese anorganische Base ist vorzugsweise eine deprotonierte anorganische Säure. Als derartige Säuren kommen insbesondere die Säuren in Betracht, die zwei und mehr Protonen abgeben können. Hierunter sind besonders Schwefel, Stickstoff oder Phosphor beinhaltende Säuren bevorzugt, wobei Schwefel oder Phosphor beinhaltende Säure drüber hinaus bevorzugt sind. Besonders haben sich Schwefel beinhaltende Säuren bewährt, hierunter insbesondere die Schwefelsäure und damit Sulfat als deren Salz für die Base. Gemäß einer besonders bevorzugten Ausführungsform des erfindungsgemäßen Verfahrens wird im Verfahrensschritt ii) als weiteres Salz ein Salz eingesetzt, welches Aluminiumsulfat beinhaltet. Vorzugsweise basiert dieses Salz zu mindestens 50 Gew.-%, darüber hinaus bevorzugt zu mindestens 75 Gew.-% und am meisten bevorzugt zu 100 Gew.-% auf Aluminiumsulfat. Die beiden verschiedenen Anionen können in einem Verhältnis von 1:100 bis 100:1, vorzugsweise in einem Verhältnis von 1 : 10 bis 10 : 1 und besonders bevorzugt von 1 : 5 bis 5 : 1 eingesetzt werden.

Weiterhin ist es erfindungsgemäß bevorzugt, dass das unbehandelte, wasserabsorbierende Polymergebilde im Verfahrensschritt ii) mit 0,001 bis 10 Gew.-%, besonders bevorzugt mit 0,01 bis 7,5 Gew.-% und am meisten bevorzugt mit 0,1 bis 5 Gew.-%, jeweils bezogen auf das Gewicht des unbehandelten, wasserabsorbierenden Polymergebildes, des Salzes oder der Salze in Kontakt gebracht wird.

Das in Kontakt bringen des Salzes mit dem unbehandelten, wasserabsorbierenden Polymergebilde im Verfahrensschritt ii) des erfindungsgemäßen Verfahrens erfolgt vorzugsweise durch Vermischen der beiden Komponenten, wobei hierzu geeignete Mischaggregate insbesondere der Patterson-Kelley-Mischer, DRAIS-Turbulenzmischer, Lödigemischer, Ruberg-Mischer, Schneckenmischer, Tellermischer und Wirbelschichtmischer oder aber kontinuierlich arbeitende senkrechte Mischer, in denen das Polymergebilde mittels rotierender Messer in schneller Frequenz gemischt wird (Schugi-Mischer), sind.

Weiterhin kann das Salz in Form eines Fluids $F_1$, umfassend ein Lösungsmittel sowie das in diesem Lösungsmittel gelöste oder dispergierte Salz, oder aber in trockener Form als Salz-Pulver mit dem unbehandelten, wasserabsorbierenden Polymergebilde in Kontakt gebracht werden. Geeignete Lösungsmittel sind, neben Wasser, insbesondere mit Wasser mischbare, organische Lösungsmittel wie etwa Methanol, Ethanol, 1-Propanol, 2-Propanol, 1,2-Propandiol, 1,3-Propandiol, 1-Butanol, 2-Butanol, tert.-Butanol, iso-Butanol oder aber Mischungen aus organischen Lösungsmitteln oder Mischungen aus Wasser mit einem oder mehreren dieser organischen Lösungsmittel, wobei Wasser als Lösungsmittel am meisten bevorzugt ist. Wird das unbehandelte, wasserabsorbierende Polymergebilde mit dem Fluid $F_1$ umfassend das Lösungsmittel und das Salz in Kontakt gebracht, so ist es weiterhin bevorzugt, dass dieses Fluid $F_1$ das Salz in einer Menge in einem Bereich von in einem Bereich von 0,1 bis 50 Gew.-%, besonders bevorzugt 1 bis 40 Gew.-% und am meisten bevorzugt 5 bis 25 Gew.-%, jeweils bezogen auf das Gesamtgewicht des Fluids $F_1$, beinhaltet.

Gemäß einer besonders bevorzugten Ausführungsform des erfindungsgemäßen Verfahrens erfolgt das in Kontakt bringen des unbehandelten, wasserabsorbierenden Polymergebildes mit dem Salz im Verfahrensschritt ii) in Gegenwart von mindestens 0,01 Gew.-% und höchstens 15 Gew.-%, besonders bevorzugt von mindestens 0,1 und höchstens 10 Gew.-%, noch mehr bevorzugt von mindestens 0,5 und höchstens 5 Gew.-%, darüber hinaus bevorzugt von mindestens 1 und höchstens 2,5 Gew.-% eines Lösungsmittels, wobei die vorstehend genannten Gew.-%-Angaben auf das Gewicht des unbehandelten, wasserabsorbierenden Polymergebildes bezogen sind.

Weiterhin kann es vorteilhaft sein, dass das in Kontakt bringen des unbehandelten, wasserabsorbierenden Polymergebilde mit dem Salz im Verfahrensschritt ii) bei einer Temperatur in einem Bereich von 30 bis 210°C, besonders bevorzugt von 50 bis 200°C und am meisten bevorzugt in einem Bereich von 160 bis 195°C erfolgt. Das in Kontakt bringen erfolgt vorzugsweise über einen Zeitraum in einem Bereich von 10 bis 300 Minuten, bevorzugt in einem Bereich von 15 bis 180 Minuten und besonders bevorzugt in einem Bereich von 20 bis 120 Minuten.

Gemäß einer besonderen Ausführungsform des erfindungsgemäßen Verfahrens zur Behandlung der Oberfläche wasserabsorbierender Polymergebilde werden im Verfahrensschritt ii) die unbehandelten, wasserabsorbierenden Polymergebilde mit dem Salz in Kontakt gebracht, wobei das Salz in Pulverform vorliegt. Das in Kontakt bringen des wasserabsorbierenden Polymergebildes mit dem Salz erfolgt in diesem Fall in Abwesenheit eines Lösungsmittels.

Bei dieser besonderen Ausführungsform des erfindungsgemäßen Verfahrens zur Herstellung wasserabsorbierender Polymergebilde umfasst das Verfahren vorzugsweise die folgenden Verfahrensschritte:

i) Bereitstellen des unbehandelten, vorzugsweise jedoch bereits oberflächennachvernetzten wasserabsorbierenden Polymergebildes;

ii) in Kontakt bringen des unbehandelten, vorzugsweise jedoch bereits oberflächennachvernetzten wasserabsorbierenden Polymergebildes mit einer Feinstteilchenkomponente umfassend das Salz bei einer Temperatur in einem Bereich von 30 bis 300°C, besonders bevorzugt von 100 bis 250°C, darüber hinaus bevorzugt in einem Bereich von 110 bis 200°C und am meisten bevorzugt in einem Bereich von 115 bis 180°C. Vorzugsweise erfolgt das in Kontakt bringen bei einer Temperatur in einem Bereich von 30 bis 200°C, darüber hinaus bevorzugt von 50 bis 160°C, darüber hinaus noch mehr bevorzugt von 50 bis 160°C und am meisten bevorzugt von 100 bis 140°C.

In diesem Zusammenhang ist es insbesondere bevorzugt, dass mindestens 50 Gew.-%, besonders bevorzugt mindestens 75 Gew.-%, darüber hinaus noch mehr bevorzugt mindestens 95 Gew.-% und am meisten bevorzugt mindestens 99 Gew.-% des in Pulverform vorliegenden Salzes einen mittleren Teilchendurchmesser (Gewichtsmittel) in einem Bereich von 10 bis 1.000 $\mu$m, vorzugsweise von 50 $\mu$m bis 800 $\mu$m, besonders bevorzugt von 100 bis 600 $\mu$m und am meisten bevorzugt von 200 bis 400 $\mu$m aufweisen, jeweils bestimmt durch dem Fachmann bekannte Verfahren zur Partikelgrößenbestimmung, beispielsweise durch Siebanalyse oder mittels eines Coulter-Counters. Weiterhin kann es bei dieser besonderen Ausführungsform des erfindungsgemäßen Verfahrens bevorzugt sein, wenn die Feinstteilchenkomponente neben dem pulverförmigen Salz zusätzlich einen Binder beinhaltet, wobei auch dieser Binder vorzugsweise in partikulärer Form vorliegt und insbesondere zu mindestens 50 Gew.-%, besonders bevorzugt zu mindestens 75 Gew.-%, darüber hinaus noch mehr bevorzugt zu mindestens 95 Gew.-% und am meisten bevorzugt zu mindestens 99 Gew.-% auf Partikel mit einem mittleren Teilchendurchmesser (Gewichtsmittel) in einem Bereich von 10 bis 1.000 $\mu$m, vorzugsweise von 50 $\mu$m bis 800 $\mu$m, besonders bevorzugt von 100 bis 600 $\mu$m und am meisten bevorzugt von 200 bis 400 $\mu$m basiert, jeweils bestimmt durch dem Fachmann bekannte Verfahren zur Partikelgrößenbestimmung, beispielsweise durch Siebanalyse oder mittels eines Coulter-Counters. Einer weiteren Ausgestaltung der vorliegenden Erfindung entspricht es, wenn das Salz als Feststoff der Nachvemetzungslösung zugegeben wird. Hierbei ist es bevorzugt, dass das Salz spätestens bei Aufgeben der Nachvernetzungslösung auf das unbehandelte, vorzugsweise noch nicht nachvemetzte, Polymergebilde in der Nachvernetzungslösung gelöst vorliegt. Die so erhaltene unbehandeltes Polymergebilde, Nachvernetzer und Salz beinhaltende Mischung wird dann der für die Nachvernetzung üblichen und hier beschriebenen Temperaturbehandlung unterzogen.

In einer erfindungsgemäßen Ausführungsform wird das Salz als vorzugsweise wässrige Lösung der Nachvernetzungslösung zugegeben. Hierdurch entstehen Nachvernetzer-Salz-Lösungen, die vorzugsweise einen Salzkonzentration im Bereich von 1 bis 50 Gew.-%, bevorzugt im Bereich von 10 bis 40 Gew.-% und besonders bevorzugt im Bereich von 15 bis 35 Gew.-%, jeweils bezogen auf die Lösungsmittelmenge der Nachvernetzungslösung. Die so erhaltene unbehandeltes Polymergebilde, Nachvernetzer und Salz beinhaltende Mischung wird dann der für die Nachvernetzung üblichen und hier beschriebenen Temperaturbehandlung unterzogen. Diese kann durch den Zusatz des nachfolgend näher erläuterten Nebensalz stablisiert werden, wo bei die nachfolgend angegebenen Mengen an Nebensalz auch hier bevorzugt sind.

In diesem Zusammenhang ist es insbesondere bevorzugt, dass der Binder als eine Binderhauptkomponente eine organische Verbindung beinhaltet, wobei die organische Verbindung vorzugsweise bei 20°C ein Feststoff ist.

Besonders bevorzugt ist es, dass die organische Verbindung ein vorzugsweise lineares Polymer ist, vorzugsweise ein lineares Polymer ausgewählt aus der Gruppe umfassend Polyurethane, Polyester, Polyamide, Polyesteramide, Polyolefine, Polyvinylester, Polyether, Polystyrole, Polyimide, insbesondere Polyetherimide, Polyimine, Schwefelpolymere, insbesondere Polysulfon, Polyacetale, insbesondere Polyoxymethylene, Fluorkunststoffe, insbesondere Polyvinylidenfluorid, Styrol-Olefin-Copolymere, Polyacrylate, Ethylen-Vinylacetat-Copolymere oder Gemische aus zwei oder mehr der genannten Polymere, wobei unter diesen Polymeren Polykondensate und unter diesen Polyether besonders bevorzugt und lineare Polyether am meisten bevorzugt sind.

Besonders geeignete lineare Polyether umfassen Polyalkylenglykole, insbesondere Polyethylenglykole, Polypropylenglykole, Poly(ethylen/propylen)glykole mit statistischer oder blockartiger Anordnung der Ethylen- oder Propylen-Monomere oder Mischungen aus mindesten zwei dieser Polyalkylenglykole.

Weitere geeignete, vorzugsweise lineare Polymere sind diejenigen Polymere, die in der DE-A-103 34 286 als "thermoplastische Klebstoffe" genannt werden.

Wenn neben dem Salz ein Binder eingesetzt wird, ist es insbesondere bevorzugt, dass das in Kontakt bringen des unbehandelten, wasserabsorbierenden Polymergebildes mit der Feinstteilchenkomponente bei einer Temperatur in einem Bereich von 30 bis 200°C, besonders bevorzugt von 50 bis 160°C und am meisten bevorzugt in einem Bereich von 70 bis 140°C erfolgt. Bei diesen Temperaturen kommt es insbesondere auch zu einer Immobilisierung der Feinstteilchen auf der Oberfläche des unbehandelten, wasserabsorbierenden Polymergebildes.

Die Menge an Bindemittel, sofern eingesetzt, liegt vorzugsweise in einem Bereich von 0,0001 bis 5 Gew.-% und besonders bevorzugt in einem Bereich von 0,001 bis 2 Gew.-%, jeweils bezogen auf das Gewicht der wasserabsorbierenden Polymergebilde. Das Gewichtsverhältnis zwischen Feinstteilchenkomponente und Bindemittel liegt vorzugsweise in einem Bereich von Feinstteilchenkomponente : Bindemittel von 20 : 1 bis 1 : 20, besonders bevorzugt von 10 : 1 bis 1 : 10 und am meisten bevorzugt von 10 : 1 bis 2 : 1.

Bei der vorstehend beschriebenen, besonderen Ausführungsform des erfindungsgemäßen Verfahrens, bei der ein pulverförmiges Salz mit den wasserabsorbierenden Polymergebilden in Kontakt gebracht wird, umfasst das Verfahren neben der Bereitstellung des unbehandelten, wasserabsorbierenden Polymergebildes im Verfahrensschritt i) auch die Bereitstellung einer Feinstteilchenkomponente umfassend das pulverförmige Salz sowie gegebenenfalls den pulverförmigen Binder. Hinsichtlich der Art und Weise des in Kontakt bringens der Feinstteilchenkomponente mit dem unbehandelten, wasserabsorbierenden Polymergebilde sind unterschiedliche Verfahrensführungen denkbar.

Weiterhin kann es im Zusammenhang mit der vorstehend beschriebenen, besonderen Ausführungsform des erfindungsgemäßen Verfahrens, bei der ein pulverfömiges Salz mit dem wasserabsorbierenden Polymergebilde in Kontakt gebracht wird, vorteilhaft sein, wenn sich an den Verfahrensschritt ii) noch ein weiterer Verfahrensschritt ii') anschließt, in dem die Mischung aus unbehandeltem, wasserabsorbierenden Polymergebilde und Feinstteilchenkomponente noch für eine Zeitraum in einem Bereich von 10 Minuten bis 5 Stunden, besonders bevorzugt von 30 Minuten bis 3 Stunden gemischt wird, um eine möglichst homogene Verteilung der Feinstteilchen bzw. der Feinstteilchenagglomerate und der absorbierenden Polymergebilde zu ermöglichen, wobei hierzu dem Fachmann bekannte Mischvorrichtungen eingesetzt werden können. In diesem weiteren Verfahrensschritt kann die Mischung aus unbehandeltem, wasserabsorbierenden Polymergebilde und Feinstteilchenkomponente mit der Temperatur, die sie nach dem Immobilisieren im Verfahrensschritt ii) aufweist, in den Mischer eingebracht werden, wobei die Mischung dann im Verlaufe des Mischens vorzugsweise stetig auf eine niedrigere Temperatur, vorzugsweise auf Raumtemperatur, abgekühlt werden kann.

Gemäß einer besonderen Ausführungsform des erfindungsgemäßen Verfahrens umfasst das Verfahren neben den Verfahrensschritten i) und ii) den Verfahrensschritt

iii) Oberflächennachvernetzung des wasserabsorbierenden Polymergebildes,

wobei der Verfahrensschritt iii) vor, während oder nach dem Verfahrensschritt ii) durchgeführt werden kann.

Bei der Oberflächennachvernetzung werden die getrockneten Polymergebilde oder aber das noch nicht getrocknete, jedoch vorzugsweise bereits zerkleinerte Hydrogel mit einem vorzugsweise organischen, chemischen Oberflächennachvernetzer in Kontakt gebracht. Dabei wird der Nachvernetzer insbesondere dann, wenn er unter den Nachvernetzungsbedingungen nicht flüssig ist, vorzugsweise in Form eines Fluids $F_2$ umfassend den Nachvernetzer sowie ein Lösungsmittel mit den Polymerteilchen bzw. dem Hydrogel in Kontakt gebracht. Als Lösungsmittel sind dabei diejenigen bevorzugt, die bereits im Zusammenhang mit dem Fluid $F_1$ als Lösungsmittel genannt wurden. Weiterhin ist es bevorzugt, dass der Nachvernetzer in dem Fluid $F_2$ in einer Menge in einem Bereich von 5 bis 75 Gew.-%, besonders bevorzugt 10 bis 50 Gew.-% und am meisten bevorzugt 15 bis 40 Gew.-%, bezogen auf das Gesamtgewicht des Fluids $F_2$, enthalten ist.

Das in Kontakt bringen des Polymergebildes bzw. des zerkleinerten Hydrogels mit dem Fluid $F_2$ beinhaltend den Nachvernetzer erfolgt im erfindungsgemäßen Verfahren vorzugsweise durch gutes Vermischen des Fluids $F_2$ mit dem Polymergebilde, wobei geeignete Mischaggregate zum Aufbringen des Fluids $F_2$ wiederum der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer), sind.

Bei der Nachvernetzung wird das wasserabsorbierende Polymergebilde vorzugsweise mit höchstens 20 Gew.-%, besonders bevorzugt mit höchstens 15 Gew.-%, darüber hinaus bevorzugt mit höchstens 10 Gew.-%, darüber hinaus noch mehr bevorzugt mit höchstens 5 Gew.-% an Lösungsmittel, vorzugsweise Wasser, jeweils bezogen auf das Gewicht des wasserabsorbierenden Polymergebildes, in Kontakt gebracht.

Bei Polymergebilden in der Form von vorzugsweise kugelförmigen Teilchen ist es erfindungsgemäß weiterhin bevorzugt, dass das in Kontakt bringen derart erfolgt, dass lediglich der Außenbereich, nicht jedoch der innere Bereich der teilchenförmigen Polymergebilde mit dem Fluid $F_2$ und somit dem Nachvernetzer in Kontakt gebracht werden.

Als Nachvernetzer, die im erfindungsgemäßen Verfahren eingesetzt werden, werden vorzugsweise Verbindungen verstanden, die mindestens zwei funktionelle Gruppen aufweisen, die mit funktionellen Gruppen eines Polymergebildes in einer Kondensationsreaktion (=Kondensationsvernetzer), in einer Additionsreaktion oder in einer Ringöffnungsreaktion reagieren können. Als Nachvernetzer sind im erfindungsgemäßen Verfahren diejenigen bevorzugt, die in WO 2004/037903 A2 als Vernetzer der Vernetzerklassen II genannt wurden.

Unter diesen Verbindungen sind als Nachvernetzer besonders bevorzugt Kondensationsvernetzer wie beispielsweise Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1, 3-dioxolan-2-on, 1,3-Dioxan-2-on, 4-Methyl-1,3-dioxan-2-on, 4,6-Dimethyl-1,3-dioxan-2-on sowie 1,3-Dioxolan-2-on.

Nachdem die Polymergebilde bzw. die Hydrogele mit dem Nachvernetzer bzw. mit dem Fluid $F_2$ beinhaltend den Nachvernetzer in Kontakt gebracht wurden, werden sie auf eine Temperatur im Bereich von 50 bis 300°C, vorzugsweise 75 bis 275°C und besonders bevorzugt 150 bis 250°C erhitzt, so dass, vorzugsweise wodurch, der Außenbereich der Polymergebilde im Vergleich zum Innenbereich stärker vernetzt wird (=Nachvernetzung). Die Zeitdauer der Wärmebehandlung wird durch die Gefahr, dass das gewünschte Eigenschaftsprofil der Polymergebilde infolge von Hitzeeinwirkung zerstört wird, begrenzt.

Die vorstehend beschriebene Oberflächenmodifizierung mittels Nachvernetzung kann vor, während oder nach dem Verfahrensschritt ii) erfolgen.

Erfolgt die Nachvernetzung vor dem Verfahrensschritt ii), so werden bereits oberflächennachvernetzte wasserabsorbierende Polymergebilde im Verfahrensschritt ii) mit dem Salz entweder in trockener Form oder aber in Form des Fluids $F_1$ in Kontakt gebracht.

Erfolgt die Nachvernetzung während des Verfahrensschrittes ii), so werden zunächst die unbehandelten, wasserabsorbierenden Polymergebilde mit dem Nachvernetzer und dem Salz

- jeweils in Abwesenheit eines Lösungsmittels,
- in Form der Fluide $F_1$ und $F_2$,
- in Form eines gemeinsamen Fluids $F_3$, umfassend das Lösungsmittel sowie den Nachvernetzer und das Salz oder aber
- das Salz in Abwesenheit eines Lösungsmittels und der Nachvernetzer in Form des Fluids $F_2$

in Kontakt gebracht und anschließend die so erhaltene Mischung zum Zwecke der Nachvernetzung auf die vorstehend genannten Temperaturen erhitzt.

Erfolgt die Nachvernetzung nach dem Verfahrensschritt ii), so werden zunächst die unbehandelten, wasserabsorbierenden Polymergebilde in der eingangs beschriebenen Art und Weise mit dem Salz, gegebenenfalls in Abwesenheit eines organischen Lösungsmittels oder aber als Fluid $F_1$, in Kontakt gebracht und anschließend die auf diese Art und Weise oberflächenmodifizierten, wasserabsorbierenden Polymergebilde oberflächennachvernetzt.

Zudem offenbart, aber nicht beansprucht wird ein wasserabsorbierendes Polymergebilde, umfassend einen Innenbereich und einen den Innenbereich umgebenden Außenbereich bei, dass zu auf mehr als 80, oder mindestens 85 und oder mindestens 90 Gew.-%, oder mehr als 95 Gew.-%, jeweils bezogen auf das wasserabsorbierende Polymergebildes, auf im Bereich von 50 bis 90 Mol-%, oder im Bereich von 60 bis 85 Mol-% oder im Bereich von 65 bis 80 Mol-% neutralisierter Acrylsäure basierend, aufweisend die folgenden Eigenschaften:

A1 einen gemäß der hierin beschriebenen Testmethode bestimmte Saline Flow Conductivity (SFC) von mehr als $115 \times 10^{-7}$ cm$^3$s/g, oder von mehr als $125 \times 10^{-7}$ cm$^3$s/g, oder von mehr als $135 \times 10^{-7}$ cm$^3$s/g oder von mehr als $145 \times 10^{-7}$ cm$^3$s/g oder mehr als $165 \times 10^{-7}$ cm$^3$s/g;

B1 eine nach ERT 442.2-02 bestimmte Absorption gegen einen Druck von 0,7 psi (AAP$_{0.7}$) von mehr als 25,9 g/g, oder von mehr als 26,4 g/g, oder von mehr als 26,9 g/g oder von mehr als 27, 5 g/g;

C1 eine nach ERT 441.2-02 bestimmte Retention (CRC) von mindestens 25 g/g, oder mindestens 27 g/g, oder mehr als 28 g/g oder mindestens 29 g/g; sowie

D1 eine Teilchengrößenverteilung mit mindestens 80 Gew.-%, oder mindestens 85 Gew.-%, oder mindestens 90 Gew.-% oder mindestens 95 Gew.-% der Teilchen des wasserabsorbierenden Polymergebildes in einem Teilchengrößenbereich von 150 bis 850 $\mu$m, wobei mindestens 5 Gew.-%, oder mindestens 8 Gew.-% oder mindestens 12 Gew.-% der Teilchen des wasserabsorbierenden Polymergebildes eine Teilchengröße in einem Bereich von 150 bis 250 $\mu$m aufweisen. Darüber hinaus ist es bevorzugt aber nicht als solches beansprucht, wenn die Menge der Teilchen des wasserabsorbierenden Polymergebildes im Bereich von 150 bis 250 $\mu$m in einem Bereich von 5 bis 30 Gew.-%, oder in einem Bereich von 7 bis 25 Gew.-% oder in einem Bereich von 10 bis 20 Gew.-% oder in einem Bereich von 11 bis 15 Gew.-%, jeweils bezogen auf die Gesamtmenge der Teilchen des wasserabsorbierenden Polymergebildes, liegen.

Gemäß einer besonderen, nicht beanspruchten Ausführungsform der wasserabsorbierenden Polymergebilde sind diese neben den vorstehend genannten Eigenschaften A1 bis D1, insbesondere neben der vorstehend genannten Eigenschaft A1, durch die folgende Eigenschaft gekennzeichnet:

E1 einen gemäß EP 0 752 892 A1 bestimmten PUP-Wert von mindestens 31 g/g, oder mindestens 32 g/g oder mindestens 33 g/g.

In diesem Zusammenhang ist es insbesondere bevorzugt aber nicht beansprucht, dass die wasserabsorbierenden Polymergebilde bei einem PUP-Wert von mindestens 31 g/g, oder von mindestens 32 g/g oder von mindestens 33 g/g eine gemäß der hierin beschriebenen Testmethode bestimmte Saline Flow Conductivity (SFC) von mehr als 100 $\times 10^{-7}$ cm$^3$s/g, aufweisen.

Ferner wird offenbart aber nicht beansprucht ein wasserabsorbierendes Polymergebilde, umfassend einen Innenbereich und einen den Innenbereich umgebenden Au-ßenbereich, dass zu auf mindestens 80, oder mindestens 90 oder mindestens 95 Gew.-%, jeweils bezogen auf das wasserabsorbierende Polymergebildes, auf im Bereich von 50 bis 90 Mol-%, oder im Bereich von 60 bis 85 Mol-% oder im Bereich von 65 bis 80 Mol-% neutralisierter Acrylsäure basierend, wobei der Au-ßenbereich des wasserabsorbierenden Polymergebildes ein Salz beinhaltend ein zwei- oder höherwertiges Kation eines Metalls beinhaltet, wobei das wasserabsorbierende Polymergebilde folgende Eigenschaften aufweist:

A2 einen gemäß der hierin beschriebenen Testmethode bestimmte Saline Flow Conductivity (SFC) von mehr als $89 \times 10^{-7}$ cm$^3$s/g, oder mehr als $115 \times 10^{-7}$ cm$^3$s/g, oder von mehr als $125 \times 10^{-7}$ cm$^3$s/g, oder von mehr als $135 \times 10^{-7}$ cm$^3$s/g oder von mehr als $145 \times 10^{-7}$ cm$^3$s/g oder mehr als $160 \times 10^{-7}$ cm$^3$s/g;

B2 eine nach ERT 442.2-02 bestimmte Absorption gegen einen Druck von 0,7 psi (AAP$_{0.7}$) von mehr als 24,7 g/g, oder von mehr als 25,9 g/g, oder von mehr als g/g 26,5, oder von mehr als 27 g/g oder von mehr als 27, 5 g/g;

C2 eine nach ERT 441.2-02 bestimmte Retention (CRC) von mindestens 25 g/g, oder mindestens 27 g/g, oder mehr als 27,2 g/g, oder mehr als 28 g/g oder mindestens 29 g/g;

D2 vorzugsweise aber nicht als solches beansprucht eine Teilchengrößenverteilung mit mindestens 80 Gew.-%, oder mindestens 85 Gew.-%, oder mindestens 90 Gew.-% oder mindestens 95 Gew.-% der Teilchen des wasserabsorbierenden Polymergebildes in einem Teilchengrößenbereich von 150 bis 850 $\mu$m, wobei mindestens 5 Gew.-%, oder mindestens 8 Gew.-% oder mindestens 12 Gew.-% der Teilchen des wasserabsorbierenden Polymergebildes eine Teilchengröße in einem Bereich von 150 bis 250 $\mu$m aufweisen. Darüber hinaus ist es bevorzugt aber nicht als solches beansprucht, wenn die Menge der Teilchen des wasserabsorbierenden Polymergebildes im Bereich von 150 bis 250 $\mu$m in einem Bereich von 5 bis 30 Gew.-%, oder in einem Bereich von 7 bis 25 Gew.-% oder in einem Bereich von 10 bis 20 Gew.-% oder in einem Bereich von 11 bis 15 Gew.-%, jeweils bezogen auf die Gesamtmenge der Teilchen des wasserabsorbierenden Polymergebildes, liegen. Gemäß einer besonderen Ausführungsform auch dieser nicht beanspruchten wasserabsorbierenden Polymergebilde sind diese neben den vorstehend genannten Eigenschaften A2 bis D2, insbesondere neben der vorstehend genannten Eigenschaft A2, durch die folgende Eigenschaft gekennzeichnet:

E2     einen gemäß EP 0 752 892 A1 bestimmten PUP-Wert von mindestens 31 g/g, oder mindestens 32 g/g odermindestens 33 g/g.

In diesem Zusammenhang ist es insbesondere bevorzugt, dass auch diese nicht beanspruchten wasserabsorbierenden Polymergebilde bei einem PUP-Wert von mindestens 31 g/g, oder von mindestens 32 g/g oder von mindestens 33 g/g eine gemäß der hierin beschriebenen Testmethode bestimmte Saline Flow Conductivity (SFC) von mehr als $100 \times 10^{-7}$ cm$^3$s/g, aufweisen.

Im Zusammenhang mit den Eigenschaften D1 und D2 entspricht es einer weiteren, nicht beanspruchtenAusgestaltung der vorliegenden Offenbarung, dass mindestens 80 Gew.-%, oder mindestens 85 Gew.-%, oder mindestens 90 Gew.-% pder mindestens 95 Gew.-% der Teilchen des wasserabsorbierenden Polymergebildes in einem Teilchengrößenbereich von 150 bis 850 μm aufweisen, wobei, jeweils bezogen auf die Menge der Teilchen mit einer Teilchengröße von 150 bis 850μm, 1 bis 20 Gew.-%, oder 5 bis 17 Gew.-% oder 7 bis 15 Gew.-% eine Teilchengröße im Bereich von 150 bis 250 μm, 15 bis 79 Gew.-%, oder 20 bis 60 Gew.-% oder 30 bis 50 Gew.-% eine Teilchengröße in einem Bereich von mehr als 250 bis 600 μm und die jeweilige restliche Menge der Teilchen zu 100 Gew.-% eine Teilchengröße in einem Bereich von mehr als 600 bis 850 μm aufweisen.

Im Zusammenhang mit den Eigenschaften D1 und D2 entspricht es einer anderen nicht beanspruchten Ausgestaltung der vorliegenden Offenbarung, dass mindestens 80 Gew.-%, oder mindestens 85 Gew.-%, oder mindestens 90 Gew.-% oder mindestens 95 Gew.-% der Teilchen des wasserabsorbierenden Polymergebildes in einem Teilchengrößenbereich von 150 bis 850 μm aufweisen, wobei, jeweils bezogen auf die Menge der Teilchen mit einer Teilchengröße von 150 bis 850μm, 1 bis 20 Gew.-%, oder 5 bis 17 Gew.-% oder 7 bis 15 Gew.-% eine Teilchengröße im Bereich von 150 bis 250 μm, 30 bis 79 Gew.-%, oder 40 bis 79 Gew.-% oder 60 bis 78 Gew.-% eine Teilchengröße in einem Bereich von mehr als 250 bis 600 μm und die jeweilige restliche Menge der Teilchen zu 100 Gew.-% eine Teilchengröße in einem Bereich von mehr als 600 bis 850 μm aufweisen.

Wie allgemein bei wasserabsorbierenden Polymeren üblich und unter anderem in DE 10 2004 005 417 A1 in Absatz [0055] beschrieben, können die nicht beanspruchten wasserabsorbierenden Polymergebilde Teilchen mit Größen aufweisen, die über den gesamten Bereich eines Teilchengrößenausschnitts liegen. In manchen Fällen liegen kleinere Mengen auch außerhalb des Bereichs von 150 bis 850 μm. So sind Teilchengrößenbereiche von 10 bis 1200 μm oder 40 bis 1000 μm oder auch 45 bis 850 μm und ebenso 100 bis 800 μm sowie 100 bis 700 μm beobachtet worden. Auch für diese Teilchengrößenbereiche bei einem offenbarten, aber nicht beanspruchten Polymergebilde gilt das zu D1 und D2 Ausgeführte entsprechend.

Einen Beitrag zur Lösung der eingangs genannten Aufgaben liefert auch ein wasserabsorbierendes Polymergebilde, welches durch das vorstehend beschriebene Verfahren erhältlich ist, wobei das wasserabsorbierende Polymergebilde einen Innenbereich und einen den Innenbereich umgebenden Außenbereich umfasst und wobei im Außenbereich des wasserabsorbierenden Polymergebildes das Salz umfassend das zwei- oder höherwertiges Kation eines Metalls sowie die mindestens eine organische Base als Anion vorhanden ist.

Offenbart aber nicht beansprucht wird außerdem ein wasserabsorbierendes Polymergebilde, umfassend einen Innenbereich und einen den Innenbereich umgebenden Außenbereich, wobei im Außenbereich des wasserabsorbierenden Polymergebildes ein Salz umfassend ein zwei- oder höherwertiges Kation eines Metalls sowie mindestens eine organische Base als Anion vorhanden ist und wobei das wasserabsorbierende Polymergebilde

A3 einen gemäß der hierin beschriebenen Testmethode bestimmte *Saline Flow Conductivity* (SFC) von mindestens $30 \times 10^{-7}$ cm$^3$s/g, oder von mindestens $50 \times 10^{-7}$ cm$^3$s/g, oder von mindestens $70 \times 10^{-7}$ cm$^3$s/g oder von mindestens $90 \times 10^{-7}$ cm$^3$s/g, ferner bevorzugt aber nicht isoliert beansprucht ein SFC-Wert von mindestens $130 \times 10^{-7}$ cm$^3$s/g oder von mindestens $155 \times 10^{-7}$ cm$^3$s/g, sowie

B3 eine nach ERT 442.2-02 bestimmte Absorption unter einem Druck (AAP) von 0,7 psi von mindestens 20 g/g, oder von mindestens 22 g/g, oder von mindestens 24 g/g oder von mindestens 26 g/g, oder von mindestens 27 g/g; aufweist.

Gemäß einer besonderen, nicht beanspruchten Ausgestaltung dieser wasserabsorbierenden Polymergebilde weisen diesen neben den beiden vorstehend genannten Eigenschaften (SFC-Wert und AAP-Wert) auch einen gemäß EP 0 752 892 A1 bestimmten PUP-Wert von mindestens 31 g/g, oder mindestens 32 g/g und oder mindestens 33 g/g auf. Auch in diesem Zusammenhang ist es insbesondere bevorzugt, dass die wasserabsorbierenden Polymergebilde bei einem PUP-Wert von mindestens 31 g/g, oder von mindestens 32 g/g oder von mindestens 33 g/g eine gemäß der hierin beschriebenen Testmethode bestimmte Saline Flow Conductivity (SFC) von mehr als $100 \times 10^{-7}$ cm³s/g, aufweisen.

Es ist möglich, die Eigenschaften wie SFC, AAP, CRC und PUP auch nach oben hin zu begrenzen. Derartige Obergrenzen liegen für den SFC in einigen Fällen bei $180 \times 10^{-7}$ cm³s/g oder erst bei $200 \times 10^{-7}$ cm³s/g und manchmal auch bei $250 \times 10^{-7}$ cm³s/g oder bei $350 \times 10^{-7}$ cm³s/g oder auch bei $500 \times 10^{-7}$ cm³s/g. Obergrenzen für den AAP liegen bei 30 g/g in manchen Fällen bei 35 g/g und gelegentlich bei 45 g/g. Obergrenzen für den CRC liegen bei 35 g/g in manchen Fällen bei 45 g/g und gelegentlich bei 50 g/g. Für den PUP-Wert liegen die Obergrenzen üblicherweise bei 40 g/g, manchmal auch bei 50 g/g und gelegentlich bei 60 g/g.

Als Salz bzw. Salze umfassend ein zwei- oder höherwertiges Kation eines Metalls sowie mindestens eine organische Base als Anion sind diejenigen Salze, Kationen und Anionen bevorzugt, die bereits eingangs im Zusammenhang mit dem erfindungsgemäßen Verfahren genannt worden sind.

Weiterhin ist es bevorzugt aber nicht isoliert beansprucht, dass das wasserabsorbierende Polymergebilde zu mindestens 50 Gew.-%, oder zu mindestens 70 Gew.-% oderzu mindestens 90 Gew.-% auf Carbonsäuregruppen-tragenden Monomeren basiert, wobei diese Carbonsäuregruppen-tragenden Monomere zu mindestens 50 Gew.-%, oder zu mindestens 70 Gew.-% aus Acrylsäure bestehen, die vorzugsweise zu mindestens 20 Mol-%, oder zu mindestens 50 Mol-% oder in einem Bereich von 60 bis 85 Mol-% neutralisiert ist.

Weiterhin sind die nicht beanspruchtenwasserabsorbierenden Polymergebilde vorzugsweise dadurch gekennzeichnet, dass der Außenbereich der wasserabsorbierenden Polymergebilde einen höheren Vernetzungsgrad aufweist als der Innenbereich der wasserabsorbierenden Polymergebilde.

Die Erfindung wird nun anhand von Figuren, Testmethoden und Beispielen näher erläutert.

Testmethoden

Bestimmung des SFC-Wertes

Die Bestimmung der Permeabilität im gequollenen Zustand (*Saline Flow Conductivity* = SFC) erfolgt nach einer in WO-A-95/22356 beschriebenen Methode. In einem Zylinder mit Siebboden werden ca. 0,9 g Superabsorbermaterial (bei Partikeln die gesamte Partikelfraktion) eingewogen und sorgfältig auf der Siebfläche verteilt. Das Superabsorbermaterial lässt man in JAYCO synthetischem Urin 1 Stunde lang gegen einen Druck von 0,7 psi quellen. Nach Erfassung der Quellhöhe des Superabsorbers lässt man bei konstantem hydrostatischem Druck 0,118 M NaCl-Lösung aus einem nivellierten Vorratsgefäß durch die gequollene Gelschicht laufen. Die gequollene Gelschicht ist während der Messung mit einem speziellen Siebzylinder abgedeckt, der eine gleichmäßige Verteilung der 0,118 M NaCl-Lösung oberhalb des Gels und konstante Bedingungen (Messtemperatur 20-25°C) während der Messung bezüglich der Gelbett-Beschaffenheit gewährleistet. Der auf den gequollenen Superabsorber wirkende Druck ist weiterhin 0,7 psi. Mit Hilfe eines Computers und einer Waage wird die Flüssigkeitsmenge, die die Gelschicht als Funktion der Zeit passiert, in Intervallen von 20 Sekunden innerhalb einer Zeitperiode von 10 Minuten erfasst. Die Fliessrate g/s durch die gequollene Gelschicht wird mittels Regressionsanalyse mit Extrapolation der Steigung und Ermittlung des Mittelpunktes auf den Zeitpunkt t=0 der Fließmenge innerhalb der Minuten 2-10 ermittelt. Der SFC-Wert (K) wurde in cm³·s·g⁻¹ angegeben und wie folgt berechnet:

$$K = \frac{F_s(t=0) \cdot L_0}{r \cdot A \cdot \Delta P_1} = \frac{F_s(t=0) \cdot L_o}{139506}$$

wobei

$F_s(t=0)$  die Fließrate in g/s,
$L_0$  die Dicke der Gelschicht in cm,
$r$  die Dichte der NaCl-Lösung (1,003 g/cm³),
$A$  die Fläche der Oberseite der Gelschicht im Messzylinder (28,27 cm²),
$\Delta P$  der hydrostatische Druck, der auf der Gelschicht lastet (4.920 dyne/cm²), und
$K$  derSFC-Wert

ist.

Bestimmung der Retention

Die als CRC bezeichnete Retention wird nach der ERT 441.2-02 bestimmt, wobei "ERT" für "EDANA recommended Test" und "EDANA" für European Disposables and Nonwovens Association" steht.

Bestimmung der Absorption unter Druck

Die als AAP bezeichnete Absorption gegen einen Druck von 0,7 psi wird nach der ERT 442.2-02 bestimmt.

Bestimmung der Teilchengröße

Die Teilchengrößen werden vorliegend nach ERT 420.2-02 bestimmt, wobei die hier angegebenen Siebe verwendet werden.

Beispiele

A. Herstellung von SAP-Partikeln

Eine Monomerlösung bestehend aus 640 g Acrylsäure, die zu 75 Mol-% mit Natronlauge neutralisiert wurde (532,82 g 50%ige NaOH), 801,32 g Wasser, 1,016 g Polyethylenglykol-300-diacrylat, 2,073 g Monoallylpolyethylenglykol-450-monoacrylsäureester wird durch Spülen mit Stickstoff vom gelösten Sauerstoff befreit und auf die Starttemperatur von 4°C abgekühlt. Nach Erreichen der Starttemperatur wurde die Initiatorlösung (0,6 g Natriumperoxydisulfat in 10 g $H_2O$, 0,014 g 35%ge Wasserstoffperoxid-Lösung in 10 g $H_2O$ und 0,03 g Ascorbinsäure in 2 g $H_2O$) zugesetzt. Nachdem die Endtemperatur von ca.100°C erreicht war, wurde das entstandene Gel mit einem Fleischwolf zerkleinert und bei 150°C 2 Stunden lang im Trockenschrank getrocknet. Das getrocknete Polymerisat wurde grob zerstoßen, mittels einer Schneidmühle SM 100 mit einem 2 mm-Konidurlochung gemahlen und auf ein Pulver mit einer Partikelgröße von 150 bis 850 $\mu$m gesiebt und so Pulver A erhalten (Korngrößen: auf 150 $\mu$m Maschenweite 13 %, auf 300 $\mu$m Maschenweite 15 %, auf 400 $\mu$m Maschenweite 13 %, auf 500 $\mu$m Maschenweite 15 %, auf 600 $\mu$m Maschenweite 20 %, auf 710 bis 850 $\mu$m Maschenweite 24 %). Die Eigenschaften dieses Pulvers sind in Tabelle 1 angegeben.

Vergleichsbeispiel 1 (herkömmliche Oberflächenmodifizierung mit Nachvernetzer und Aluminiumsulfat)

100 g des Pulvers A werden mit einer Lösung bestehend aus 1,0 g Ethylencarbonat (EC), 0,6 g $Al_2(SO_4)_3 \times 14$ $H_2O$ und 3 g entionisiertes Wasser vermischt, wobei die Lösung mittels einer Spritze mit einer 0,45 mm-Kanüle auf das sich in einem Mischer befindliche Polymerisatpulver aufgetragen wurde. Anschließend wurde das mit der wässrigen Lösung beschichtete Pulver A in einem Umluftschrank bei 185°C für 30 Minuten erhitzt. Es wurde ein Pulver A1 erhalten (Korngrößen: auf 150 $\mu$m Maschenweite 13 %, auf 300 $\mu$m Maschenweite 15 %, auf 400 $\mu$m Maschenweite 12 %, auf 500 $\mu$m Maschenweite 15 %, auf 600 $\mu$m Maschenweite 20 %, auf 710 bis 850 $\mu$m Maschenweite 25 %). Die Eigenschaften dieses Pulvers sind in Tabelle 1 angegeben.

Beispiel 1 (erfindungsgemäße Oberflächenmodifizierung mit Lösung aus Nachvernetzer und Aluminiumlactat)

100 g des Pulvers A werden mit einer Lösung bestehend aus 1,0 g Ethylencarbonat, 0,6 g Aluminiumlactat und 3 g entionisiertes Wasser vermischt, wobei die Lösung mittels einer Spritze mit einer 0,45 mm-Kanüle auf das sich in einem Mischer befindliche Polymerisatpulver aufgetragen wurde. Anschließend wurde das mit der wässrigen Lösung beschichtete Pulver A in einem Umluftschrank bei 185°C für 30 Minuten erhitzt. Es wurde ein Pulver A2 erhalten (Korngrößen: auf 150 $\mu$m Maschenweite 12 %, auf 300 $\mu$m Maschenweite 16 %, auf 400 $\mu$m Maschenweite 14 %, auf 500 $\mu$m Maschenweite 14 %, auf 600 $\mu$m Maschenweite 21 %, auf 710 bis 850 $\mu$m Maschenweite 23 %). Die Eigenschaften dieses Pulvers sind in Tabelle 1 angegeben.

Beispiel 2 (erfindungsgemäße Oberflächenmodifizierung mit Nachvernetzer und Aluminiumlactat vor Nachvernetzung fest)

100 g des Pulvers A werden 0,6 g Aluminiumlactatpulver (Korngröße: 85 % im Bereich von 45 bis 150 $\mu$m, >150 $\mu$m 9 %, <45 $\mu$m 6 %) über 10 Minuten innig vermischt und mit einer Lösung bestehend aus 1,0 g Ethylencarbonat, und 3 g entionisiertes Wasser vermischt, wobei die Lösung mittels einer Spritze mit einer 0,45 mm-Kanüle auf das sich in einem Mischer befindliche Polymerisatpulver aufgetragen wurde. Anschließend wurde das mit der wässrigen Lösung beschichtete Pulver A in einem Umluftschrank bei 185°C für 30 Minuten erhitzt. Es wurde ein Pulver A3 erhalten (Korn-

größen: auf 150 μm Maschenweite 14 %, auf 300 μm Maschenweite 14 %, auf 400 μm Maschenweite 13 %, auf 500 μm Maschenweite 15 %, auf 600 μm Maschenweite 19 %, auf 710 bis 850 μm Maschenweite 25 %).

Beispiel 3 (erfindungsgemäße Oberflächenmodifizierung mit Nachvernetzer und Aluminiumlactat nach Nachvernetzung fest)

100 g des Pulvers A werden mit einer Lösung bestehend aus 1,0 g Ethylencarbonat, und 3 g entionisiertes Wasser vermischt, wobei die Lösung mittels einer Spritze mit einer 0,45 mm-Kanüle auf das sich in einem Mischer befindliche Polymerisatpulver aufgetragen wurde. Anschließend wurde das mit der wässrigen Lösung beschichtete Pulver A in einem Umluftschrank bei 185°C für 30 Minuten erhitzt. Anschließend wurde dieses Produkt 0,6 g Aluminiumlactatpulver (Korngröße: 85 % im Bereich von 45 bis 150 μm, >150 μm 9 %, <45 μm 6 %) über 10 Minuten innig vermischt. Es wurde ein Pulver A4 erhalten (Korngrößen: auf 150 μm Maschenweite 15 %, auf 300 μm Maschenweite 14 %, auf 400 μm Maschenweite 13 %, auf 500 μm Maschenweite 14 %, auf 600 μm Maschenweite 19 %, auf 710 bis 850 μm Maschenweite 25 %).

Beispiel 4 (erfindungsgemäße Oberflächenmodifizierung mit Nachvernetzer und Aluminiumlactat nach Nachvernetzung flüssig)

100 g des Pulvers A werden mit einer Lösung bestehend aus 1,0 g Ethylencarbonat, und 3 g entionisiertes Wasser vermischt, wobei die Lösung mittels einer Spritze mit einer 0,45 mm-Kanüle auf das sich in einem Mischer befindliche Polymerisatpulver aufgetragen wurde. Anschließend wurde das mit der wässrigen Lösung beschichtete Pulver A in einem Umluftschrank bei 170°C für 30 Minuten erhitzt. Anschließend wurde dieses Produkt mit einer Lösung aus 0,6 g Aluminiumlactat in 3 g entionisierten Wasser über 30 Minuten vorsichtig zu Pulver innig vermischt. Dieses Mischungsprodukt wurde bei 130°C für 30 Minuten getrocknet und so Pulver A5 erhalten (Korngrößen: auf 150 μm Maschenweite 12 %, auf 300 μm Maschenweite 15 %, auf 400 μm Maschenweite 14 %, auf 500 μm Maschenweite 15 %, auf 600 μm Maschenweite 18 %, auf 710 bis 850 μm Maschenweite 26 %).

Beispiel 5 (erfindungsgemäße Oberflächenmodifizierung mit Lösung aus Nachvernetzer Aluminiumlactat und -sulfat)

100 g des Pulvers A werden mit einer Lösung bestehend aus 1,0 g Ethylencarbonat, 0,2 g Aluminiumlactat, 0,4 g Aluminiumsulfat und 3 g entionisiertes Wasser vermischt, wobei die Lösung mittels einer Spritze mit einer 0,45 mm-Kanüle auf das sich in einem Mischer befindliche Polymerisatpulver aufgetragen wurde. Anschließend wurde das mit der wässrigen Lösung beschichtete Pulver A in einem Umluftschrank bei 185°C für 30 Minuten erhitzt. Es wurde ein Pulver A6 erhalten (Korngrößen: auf 150 μm Maschenweite 12 %, auf 300 μm Maschenweite 15 %, auf 400 μm Maschenweite 14 %, auf 500 μm Maschenweite 15 %, auf 600 μm Maschenweite 20 %, auf 710 bis 850 μm Maschenweite 24 %). Die Eigenschaften dieses Pulvers sind in Tabelle 1 angegeben.

Beispiel 6 (erfindungsgemäße Oberflächenmodifizierung mit Lösung aus Nachvernetzer Aluminiumlactat und -sulfat)

100 g des Pulvers A werden mit einer Lösung bestehend aus 1,0 g Ethylencarbonat, 0,3 g Aluminiumlactat, 0,3 g Aluminiumsulfat und 3 g entionisiertes Wasser vermischt, wobei die Lösung mittels einer Spritze mit einer 0,45 mm-Kanüle auf das sich in einem Mischer befindliche Polymerisatpulver aufgetragen wurde. Anschließend wurde das mit der wässrigen Lösung beschichtete Pulver A in einem Umluftschrank bei 190°C für 30 Minuten erhitzt. Es wurde ein Pulver A7 erhalten (Korngrößen: auf 150 μm Maschenweite 12 %, auf 300 μm Maschenweite 15 %, auf 400 μm Maschenweite 13 %, auf 500 μm Maschenweite 15 %, auf 600 μm Maschenweite 20 %, auf 710 bis 850 μm Maschenweite 25 %). Die Eigenschaften dieses Pulvers sind in Tabelle 1 angegeben.

Tabelle 1

| Pulver | Beschichtung mit | | | Eigenschaften | | |
|--------|------------------|------------------|------------------|-----------|-------------------|-------|
|        | EC [Gew.-%]      | Al-sulfat [Gew.-%] | Al-lactat [Gew.-%] | CRC [g/g] | AAP (0,7 psi) [g/g] | SFC** |
| A      | 0                | 0                | 0                | 33,8      | 20*               |       |
| A1     | 1,0              | 0,6              | 0                | 29,6      | 23,7              | 54    |
| A2     | 1,0              | 0                | 0,6              | 29,7      | 25,3              | 74    |
| A6     | 1,0              | 0,4              | 0,2              | 28,9      | 24,0              | 101   |

(fortgesetzt)

| Pulver | Beschichtung mit | | | Eigenschaften | | |
|---|---|---|---|---|---|---|
| | EC [Gew.-%] | Al-sulfat [Gew.-%] | Al-lactat [Gew.-%] | CRC [g/g] | AAP (0,7 psi) [g/g] | SFC** |
| A7 | 1,0 | 0,3 | 0,3 | 28,2 | 24,6 | 110 |
| * bei 0,3 psi bestimmt.<br>** [x $10^{-7}$ cm$^3$s/g] | | | | | | |

B. Herstellung von SAP-Partikeln

Eine Monomerlösung bestehend aus 260 g Acrylsäure, die zu 70 Mol-% mit Natronlauge neutralisiert wurde (202,054 g 50%ige NaOH), 505,899 g Wasser, 0,409 g Polyethylenglykol-300-diacrylat, 1,253 g Monoallylpolyethylenglykol-450-monoacrylsäureester wird durch Spülen mit Stickstoff vom gelösten Sauerstoff befreit und auf die Starttemperatur von 4°C abgekühlt. Nach Erreichen der Starttemperatur wurde die Initiatorlösung (0,3 g Natriumperoxydisulfat in 10 g $H_2O$, 0,07 g 35%ge Wasserstoffperoxid-Lösung in 10 g $H_2O$ und 0,015 g Ascorbinsäure in 2 g $H_2O$) zugesetzt. Nachdem die Endtemperatur von ca. 100°C erreicht war, wurde das entstandene Gel mit einem Fleischwolf zerkleinert und bei 150°C 2 Stunden lang im Trockenschrank getrocknet. Das getrocknete Polymerisat wurde grob zerstoßen, mittels einer Schneidmühle SM 100 mit einem 2 mm-Konidurlochung gemahlen und auf zwei Pulver B und C mit einer Partikelgröße von 150 bis 850 μm gesiebt, die die in Tabelle 2 angegebenen Korngrößen aufweisen.

Tabelle 2

| Pulver | 1150 bis 250μm [%] | >250 bis 600 μm [%] | >600 bis 850 μm [%] |
|---|---|---|---|
| B | 8 | 42 | 50 |
| C | 12,5 | 75 | 12,5 |

Vergleichsbeispiel 2 (herkömmliche Oberflächenmodifizierung mit Nachvernetzer und Aluminiumsulfat)

100 g des Pulvers B werden mit einer Lösung bestehend aus 1,0 g Ethylencarbonat (EC), 0,6 g $Al_2(SO_4)_3 \times$ 14 $H_2O$ und 3 g entionisiertes Wasser vermischt, wobei die Lösung mittels einer Spritze mit einer 0,45 mm-Kanüle auf das sich in einem Mischer befindliche Polymerisatpulver aufgetragen wurde. Anschließend wurde das mit der wässrigen Lösung beschichtete Pulver B in einem Umluftschrank bei 170°C für 90 Minuten erhitzt. Es wurde ein Pulver B1 erhalten (Korngrößen: auf 150 μm Maschenweite 7 %, auf 250 μm Maschenweite 43 %, auf 600 bis 850 μm Maschenweite 50 %). Die Eigenschaften dieses Pulvers sind in Tabelle 3 angegeben.

Vergleichsbeispiel 3 (herkömmliche Oberflächenmodifizierung mit Nachvernetzer und Aluminiumsulfat)

Vergleichsbeispiel 2 wurde mit dem Unterschied wiederholt, dass anstelle von Pulver B das Pulver C eingesetzt wurde. So wurde Pulver C1 erhalten (Korngrößen: auf 150 μm Maschenweite 12 %, auf 250 μm Maschenweite 75,5 %, auf 600 bis 850 μm Maschenweite 12,5 %). Die Eigenschaften dieses Pulvers sind in Tabelle 4 angegeben.

Beispiel 7 (erfindungsgemäße Oberflächenmodifizierung mit Lösung aus Nachvernetzer und festem Aluminiumlactat)

100 g des Pulvers B werden mit einer Lösung bestehend aus 1,0 g Ethylencarbonat, 0,6 g festes Aluminiumlactat und 3 g entionisiertes Wasser vermischt, wobei die Lösung mittels einer Spritze mit einer 0,45 mm-Kanüle auf das sich in einem Mischer befindliche Polymerisatpulver aufgetragen wurde. Anschließend wurde das mit der wässrigen Lösung beschichtete Pulver B in einem Umluftschrank bei 185°C für 30 Minuten erhitzt. Es wurde ein Pulver B2 erhalten (Korngrößen: auf 150 μm Maschenweite 9 %, auf 250 μm Maschenweite 40 %, auf 600 bis 850 μm Maschenweite 51 %). Die Eigenschaften dieses Pulvers sind in Tabelle 3 angegeben.

Beispiel 8 (erfindungsgemäße Oberflächenmodifizierung mit Lösung aus Nachvernetzer und festem Aluminiumlactat)

Beispiel 7 wurde mit dem Unterschied wiederholt, dass anstelle von Pulver B das Pulver C eingesetzt wurde. So wurde Pulver C2 erhalten (Korngrößen: auf 150 μm Maschenweite 11,5 %, auf 300 μm Maschenweite 76 %, auf 600 bis 850 μm Maschenweite 12,5 %). Die Eigenschaften dieses Pulvers sind in Tabelle 4 angegeben.

Beispiel 9 (erfindungsgemäße Oberflächenmodifizierung mit Lösung aus Nachvernetzer und Aluminiumlactatlösung)

100 g des Pulvers B werden mit einer ersten Lösung bestehend aus 1,0 g Ethylencarbonat und 1,457 g entionisiertem Wasser und 0,6 g Aluminumlactat in Form einer 28 %igen mit NaOH stabilisierten Aluminiumlactatlösung als zweite Lösung vermischt, wobei die Lösungen mittels einer Spritze mit einer 0,45 mm-Kanüle auf das sich in einem Mischer befindliche Polymerisatpulver aufgetragen wurde. Zur Herstellung der NaOH stabilisierten Aluminiumlactatlösung werden 0,0625 Mol (7,38 g) Natriumaluminat in 15 ml Wasser gelöst, gefolgt von 0,0475 Mol NaOH (2,5 ml einer 50 %igen wässrigen NaOH-Lösung), wobei darauf zu dieser Lösung eine Mischung einer 85% Milchsäure, in 20 ml Wasser verdünnt, langsam zugegeben wird, bis ein pH-Wert von 4,5 erreicht wird (0,29 mol Milchsäure = 31,0 g). Anschließend wurde das mit den wässrigen Lösungen beschichtete Pulver B in einem Umluftschrank bei 185°C für 30 Minuten erhitzt. Es wurde ein Pulver B3 erhalten (Korngrößen: auf 150 $\mu$m Maschenweite 9 %, auf 250 $\mu$m Maschenweite 40 %, auf 600 bis 850 $\mu$m Maschenweite 51 %). Die Eigenschaften dieses Pulvers sind in Tabelle 3 angegeben.

Beispiel 10 (erfindungsgemäße Oberflächenmodifizierung mit Lösung aus Nachvernetzer und Aluminiumlactatlösung)

Beispiel 9 wurde mit dem Unterschied wiederholt, dass anstelle von Pulver B das Pulver C eingesetzt wurde. So wurde Pulver C3 erhalten (Korngrößen: auf 150 $\mu$m Maschenweite 11,5 %, auf 250 $\mu$m Maschenweite 76 %, auf 600 bis 850 $\mu$m Maschenweite 12,5 %). Die Eigenschaften dieses Pulvers sind in Tabelle 4 angegeben.

Beispiel 11

100 g des Pulvers B werden 0,6 g Aluminiumlactatpulver (Korngröße: 85 % im Bereich von 45 bis 150 $\mu$m, >150 $\mu$m 9 %, <45 $\mu$m 6 %) über 10 Minuten innig vermischt und mit einer Lösung bestehend aus 1,0 g Ethylencarbonat, und 3 g entionisiertes Wasser vermischt, wobei die Lösung mittels einer Spritze mit einer 0,45 mm-Kanüle auf das sich in einem Mischer befindliche Polymerisatpulver aufgetragen wurde. Anschließend wurde das mit der wässrigen Lösung beschichtete Pulver B in einem Umluftschrank bei 170°C für 90 Minuten erhitzt. Es wurde ein Pulver B4 erhalten (Korngrößen: auf 150 $\mu$m Maschenweite 14 %, auf 300 $\mu$m Maschenweite 13 %, auf 400 $\mu$m Maschenweite 13 %, auf 500 $\mu$m Maschenweite 15 %, auf 600 $\mu$m Maschenweite 19 %, auf 710 bis 850 $\mu$m Maschenweite 25 %).

Beispiel 12

Beispiel 8 wurde mit dem Unterschied wiederholt, dass anstelle von Pulver B das Pulver C eingesetzt wurde. So wurde Pulver C4 erhalten (Korngrößen: auf 150 $\mu$m Maschenweite 11,5 %, auf 300 $\mu$m Maschenweite 76 %, auf 600 $\mu$m Maschenweite 12,5 %).

Tabelle 3

| Pulver | Beschichtung mit | | | Eigenschaften | | |
|---|---|---|---|---|---|---|
| | EC [Gew.-%] | Al-sulfat [Gew.-%] | Al-lactat [Gew.-%] | CRC [g/g] | AAP (0,7 psi) [g/g] | SFC* |
| B | 0 | 0 | 0 | 30,5 | 26,2 | 57 |
| B1 | 1,0 | 0,6 | 0 | 29,5 | 25,0 | 90 |
| B2 | 1,0 | 0 | 0,6 (Feststoff) | 29,5 | 26,8 | 159 |
| B3 | 1,0 | 0 | 0,6 (Lösung) | 29,1 | 27,8 | 160 |
| *[$\times$ 10$^{-7}$ cm$^3$s/g] | | | | | | |

Tabelle 4

| Pulver | Beschichtung mit | | | Eigenschaften | | |
|---|---|---|---|---|---|---|
| | EC [Gew.-%] | Al-sulfat [Gew.-%] | Al-lactat [Gew.-%] | CRC [g/g] | AAP (0,7 psi) [g/g] | SFC* |
| C | 0 | 0 | 0 | 30,8 | 27,5 | 48 |
| C1 | 1,0 | 0,6 | 0 | 29,1 | 24,9 | 115 |
| C2 | 1,0 | 0 | 0,6 (Feststoff) | 28,8 | 27,6 | 134 |

(fortgesetzt)

| Pulver | Beschichtung mit | | | Eigenschaften | | |
|---|---|---|---|---|---|---|
| | EC [Gew.-%] | Al-sulfat [Gew.-%] | Al-lactat [Gew.-%] | CRC [g/g] | AAP (0,7 psi) [g/g] | SFC* |
| C3 | 1,0 | 0 | 0,6 (Lösung) | 29,1 | 24,9 | 160 |
| * [$\times$ 10$^{-7}$ cm$^3$s/g] | | | | | | |

C. Herstellung von SAP-Partikeln

Die Herstellung von SAP-Partikeln gemäß Beispiel B wurde wiederholt, wobei zwei Pulver D und E erhalten wurden, die die in der Tabelle 5 angegebenen Korngrößen aufweisen:

Tabelle 5

| Pulver | 150 bis 300 $\mu$m [%] | >300 bis 600 $\mu$m [%] | >600 bis 850 $\mu$m [%] |
|---|---|---|---|
| D | 13 | 37 | 50 |
| E | 12,5 | 75 | 12,5 |

Beispiel 13

100 g des Pulvers E werden wie im Beispiel 7 beschrieben behandelt, wobei jedoch im Unterschied zum Beispiel 7 bei 170°C für 90 Minuten erhitzt wurde. Es wurde ein Pulver E1 erhalten. Die Eigenschaften dieses Pulvers sind in Tabelle 6 angegeben.

Beispiel 14

100 g des Pulvers E werden wie im Beispiel 9 beschrieben behandelt, wobei jedoch im Unterschied zum Beispiel 9 bei 170°C für 90 Minuten erhitzt wurde. Es wurde ein Pulver E2 erhalten. Die Eigenschaften dieses Pulvers sind in Tabelle 6 angegeben.

Tabelle 6

| Pulver | Beschichtung mit | | | Eigenschaften | | |
|---|---|---|---|---|---|---|
| | EC [Gew.-%] | Al-sulfat [Gew.-%] | Al-lactat [Gew.-%] | CRC [g/g] | PUP [g/g] | SFC* |
| E | 0 | 0 | 0 | 29,0 | 33,3 | 71 |
| E1 | 1,0 | 0 | 0,6 (Feststoff) | 28,9 | 32,8 | 104 |
| E2 | 1,0 | 0 | 0,6 (Lösung) | 29,1 | 32,4 | 148 |
| * [x 10$^{-7}$ cm$^3$s/g] | | | | | | |

Beispiel 15

100 g des Pulvers D werden wie im Beispiel 7 beschrieben behandelt, wobei jedoch im Unterschied zum Beispiel 7 bei 170°C für 90 Minuten erhitzt wurde. Es wurde ein Pulver D1 erhalten. Die Eigenschaften dieses Pulvers sind in Tabelle 7 angegeben.

Beispiel 16

100 g des Pulvers D werden wie im Beispiel 9 beschrieben behandelt, wobei jedoch im Unterschied zum Beispiel 9 bei 170°C für 90 Minuten erhitzt wurde. Es wurde ein Pulver D2 erhalten. Die Eigenschaften dieses Pulvers sind in Tabelle 7 angegeben.

Tabelle 7

| Pulver | Beschichtung mit | | | Eigenschaften | | |
|--------|------------------|--|--|---------------|--|--|
|        | EC [Gew.-%] | Al-sulfat [Gew.-%] | Al-lactat [Gew.-%] | CRC [g/g] | PUP [g/g] | SFC* |
| D | 0 | 0 | 0 | 29,4 | 33,1 | 68 |
| D1 | 1,0 | 0 | 0,6 (Feststoff) | 29,6 | 33,3 | 131 |
| D2 | 1,0 | 0 | 0,6 (Lösung) | 29,1 | 33,1 | 176 |
| * [× $10^{-7}$ cm$^3$s/g] LEERSEITE | | | | | | |

**Patentansprüche**

**1.** Ein Verfahren zur Herstellung eines wasserabsorbierenden Polymergebildes, umfassend die Verfahrensschritte:

i) Bereitstellen eines unbehandelten, wasserabsorbierenden Polymergebildes;
ii) in Kontakt bringen des unbehandelten, wasserabsorbierenden Polymergebildes mit Aluminiumlactat.

**2.** Verfahren nach einem der vorhergehenden Ansprüche, wobei das unbehandelte, wasserabsorbierende Polymergebilde im Verfahrensschritt ii) mit 0,001 bis 10 Gew.-%, bezogen auf das Gewicht des unbehandelten, wasserabsorbierenden Polymergebildes, des Salzes in Kontakt gebracht wird.

**3.** Verfahren nach einem der vorhergehenden Ansprüche, wobei das in Kontakt bringen des unbehandelten, wasserabsorbierenden Polymergebildes mit dem Salz im Verfahrensschritt ii) in Gegenwart von höchstens 15 Gew.-%, bezogen auf das Gewicht des unbehandelten, wasserabsorbierenden Polymergebildes, eines Lösungsmittels erfolgt.

**4.** Verfahren nach einem der vorhergehenden Ansprüche, wobei das Verfahren neben den Verfahrensschritten i) und ii) den Verfahrensschritt

iii) Oberflächennachvernetzung des wasserabsorbierenden Polymergebildes
umfasst und wobei der Verfahrensschritt iii) vor, während oder nach dem Verfahrensschritt ii) durchgeführt werden kann.

**5.** Ein wasserabsorbierendes Polymergebilde, erhältlich durch ein Verfahren nach einem der Ansprüche 1 bis 4, wobei das wasserabsorbierende Polymergebilde einen Innenbereich und einen den Innenbereich umgebenden Außenbereich umfasst und wobei im Außenbereich des wasserabsorbierenden Polymergebildes ein Aluminiumlactat vorhanden ist.

**Claims**

**1.** Process for producing a water-absorbing polymer structure, comprising the process steps of:

i) providing an untreated, water-absorbing polymer structure;
ii) contacting the untreated, water-absorbing polymer structure with aluminium lactate.

**2.** Process according to any of the preceding claims, wherein the untreated, water-absorbing polymer structure is contacted in process step ii) with 0.001% to 10% by weight, based on the weight of the untreated water-absorbing polymer structure, of the salt.

**3.** Process according to any of the preceding claims, wherein the contacting of the untreated water-absorbing polymer structure with the salt in process step ii) is effected in the presence of not more than 15% by weight, based on the weight of the untreated, water-absorbing polymer structure, of a solvent.

**EP 2 012 843 B1**

4. Process according to any of the preceding claims, wherein the process comprises, as well as process steps i) and ii), the process step of iii) surface postcrosslinking of the water-absorbing polymer structure, and wherein process step iii) can be performed before, during or after process step ii).

5. Water-absorbing polymer structure obtainable by a process according to any of Claims 1 to 4, wherein the water-absorbing polymer structure comprises an inner region and an outer region that surrounds the inner region, and wherein an aluminium lactate is present in the outer region of the water-absorbing polymer structure.

**Revendications**

1. Procédé pour la production d'une structure polymère absorbant l'eau, comprenant les étapes de procédé consistant à :

    i) fournir une structure polymère absorbant l'eau, non traitée ;
    ii) mettre la structure polymère absorbant l'eau, non traitée, en contact avec du lactate d'aluminium.

2. Procédé selon l'une quelconque des revendications précédentes, dans lequel la structure polymère absorbant l'eau, non traitée, est mise en contact, dans l'étape ii) du procédé, avec 0,001 à 10 % en poids du sel, par rapport au poids de la structure polymère absorbant l'eau, non traitée.

3. Procédé selon l'une quelconque des revendications précédentes, dans lequel la mise en contact de la structure polymère absorbant l'eau, non traitée, avec le sel dans l'étape ii) du procédé s'effectue en présence d'au maximum 15 % en poids d'un solvant, par rapport au poids de la structure polymère absorbant l'eau, non traitée.

4. Procédé selon l'une quelconque des revendications précédentes, dans lequel le procédé comprend, outre les étapes i) et ii) du procédé, l'étape de procédé iii) post-réticulation superficielle de la structure polymère absorbant l'eau et dans lequel l'étape iii) du procédé peut être effectuée avant, pendant ou après l'étape ii) du procédé.

5. Structure polymère absorbant l'eau, pouvant être obtenue par un procédé selon l'une quelconque des revendications 1 à 4, dans laquelle la structure polymère absorbant l'eau comprend une zone interne et une zone externe entourant la zone interne et dans laquelle un lactate d'aluminium est présent dans la zone externe de la structure polymère absorbant l'eau.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE OS2612846 A
- DE 19646484
- DE 19909653 A1
- DE 19909838 A1
- DE 10249821 A1
- DE 19529348 A1
- WO 9934843 A1
- WO 2004037903 A2
- US 4286082 A
- DE 2706135
- US 4076663 A
- DE 3503458
- DE 4020780
- DE 4244548
- DE 4323001
- DE 4333056
- DE 4418818
- US 4340706 A
- DE 3713601
- DE 2840010
- WO 9605234 A1
- DE 10334286 A
- EP 0752892 A1
- DE 102004005417 A1
- WO 9522356 A